# EUROPEAN PATENT APPLICATION

(11) **EP 0 633 242 A1**
(43) Date of publication of application: **11.01.1995**
(21) Application number: 94401534.6
(22) Date of filing: 05.07.1994
(51) Int. Cl.: C07C 69/716, C07D 319/06

(54) **Heptanoate derivatives**

(30) Priority: 05.07.1993 JP 190945/93
(71) Applicant: FUJIREBIO Inc., Shinjuku-ku, Tokyo 163-07 (JP)
(72) Inventor: Matsumoto, Masakatsu, Sagamihara-shi, Kanagawa-ken (JP); Watanabe, Nobuko, c/o Fujirebio Inc., Tokyo (JP); Mori, Eiko, c/o Fujirebio Inc., Tokyo (JP); Kobayashi, Hisako, c/o Fujirebio Inc., Tokyo (JP); Ikawa, Hiroshi, c/o Fujirebio Inc., Tokyo (JP)
(74) Representative: Gillard, Marie-Louise

(57) **Abstract**

A heptanoate derivative of formula (I): 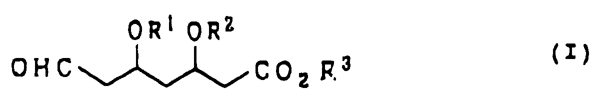
wherein R¹ and R² are independently a hydrogen atom or a protective group for hydroxyl group, or R¹ and R² integrally constitute a protective group for hydroxyl groups; and R³ is a hydrogen atom, an alkyl group, or an aromatic hydrocarbon group.

## Description

### BACKGROUND OF THE INVENTION

### Field of the Invention

The present invention relates to a heptanoate derivative represented by the following general formula (I), which can be useful as an intermediate for producing an anti-hypercholesterolemic agent, which has an inhibitory effect on, for instance, 3-hydroxy-3-methylglutaryl-CoA reductase (hereinafter referred to as HMG-CoA Reductase): 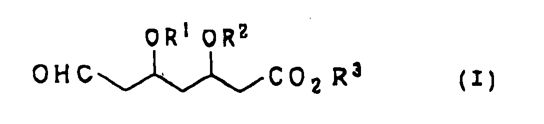
wherein R¹ and R² are independently a hydrogen atom or a protective group for hydroxyl group, or R¹ and R² taken together constitute a protective group for hydroxyl groups; and R³ is a hydrogen atom, an alkyl group which may be substituted , or an aromatic hydrocarbon group.

### Discussion of Background

Compounds having as a substituent an (E)-3,5-dihydroxy-6-heptenoic acid moiety which is bonded thereto at the 7-position thereof, which are hereinafter referred to as 7-position-substituted (E)-3,5-dihydroxy-6-heptenoic acid compounds, serve as HMG-CoA Reductase inhibitors. Many methods of synthesizing such 7-position-substituted (E)-3,5-dihydroxy-6-heptenoic acid compounds, including the methods of synthesizing optical active compounds thereof, have been reported.

The substituent at the 7-position of the (E)-3,5-dihydroxy-6-heptenoic acid moiety of the above-mentioned 7-position-substituted (E)-3,5-dihydroxy-6-heptenoic acid compounds (hereinafter referred to the 7-position substituent) is a key substituent for the generation of the inhibitory activity against the HMG-CoA Reductase. Examples of such a 7-position substituent include an aromatic group, heterocyclic group, and a substituted vinyl group.

Methods of introducing the 3,5-dihydroxy-6-heptenoic acid unit into this 7-position substituent can be roughly classified into the following three methods:
(I) A method of introducing a C3 carbon chain into the 7-position substituent, followed by the introduction of a C4 carbon chain.
   This method includes, for instance, the following specific methods:
   (I-1) A method of introducing as the C4 carbon chain a β-keto ester (J. Med. Chem. 1986, 29, 170, J. Org. Chem. 1991, 56, 5752).
   (I-2) A method of introducing as the C4 carbon chain a C2 carbon chain for asymmetric induction, followed by the introduction of a C2 carbon chain (Tetrahedron Lett, 1984, 25, 5031).
   (I-3) A method of introducing as the C4 carbon chain an optical active C4 carbon chain which is derived from isoascorbic acid through complicated steps (Tetrahedron Lett, 1985, 26, 2951).
(II) A method of introducing a C1 carbon chain into the 7-position substituent structure, followed by the introduction of a C6 carbon chain.
   This method includes, for instance, the following specific methods:
   (II-1) A method of allowing a formyl group introduced into the 7-position substituent to react with an optically active Horner-Emmons Reagent having a C6 carbon chain (J. Med. Chem. 1987, 30, 1858).
   (II-2) A method of utilizing a substituted sulfinylmethyl group introduced into the 7-position substituent (Tetrahedron Lett. 1985, 25, 2947).
   (II-3) A method of utilizing the Wittig reagent or Horner-Emmons reagent converted from the halomethyl group introduced into the 7-position substituent (Tetrahedron Lett. 1982, 23, 4305, Tetrahedron Lett. 1990, 31, 2545).
(III) A method of directly introducing a C7 carbon chain in the 7-position substituent or utilizing a halide compound serving as the 7-position substituent.
   This method includes, for instance, the following specific methods:
   (III-1) A method of utilizing Heck Reaction employing a C7 carbon chain having a carbon-carbon unsaturated bond at the terminal thereof (J. Med. Chem. 1990, 33, 31).
   (III-2) A method of utilizing the reaction between a silyl-substituted epoxy compound having a C7 carbon chain and an anion species prepared from a halide compound (Tetrahedron Lett. 1992, 33, 4183).
   (III-3) A method of utilizing Heck type reaction between a tin-olefin compound synthesized from the above-mentioned silyl-substituted epoxy compound having a C7 carbon chain and a halide compound (Tetrahedron Lett. 1992, 33, 4183).

None of the above-mentioned methods, however, is suitable for industrial production of the 7-position-substituted (E)-3,5-dihydroxy-6-heptenoic acid compounds because of the following respective shortcomings:

In the methods (I-1), (1-2) and (I-3), and (II-1), a reduction reaction which is apt to cause problems with respect to the reproducibility thereof is required at a stage close to the final stage of the synthesis of the desired product.

The method (II-2) is complicated because it includes a number of steps after the introduction of the C6 carbon chain.

The method (II-3) not only requires multi-stage steps for synthesizing Wittig reagent and Horner-Emmons reagent, but also has the problems with respect to the yields of the products obtained by Wittig reaction and Horner-Emmons reaction.

The methods (III-1) and (III-3) require an expensive palladium catalyst.

The method (III-2) requires complicated steps for producing the silyl-substituted epoxy compound to be employed therein.

### SUMMARY OF THE INVENTION

It is therefore an object of the present invention to provide a heptanoate derivative which can be produced easily with high yield.

This object of the present invention can be achieved by a heptanoate derivative of the following general formula (I): 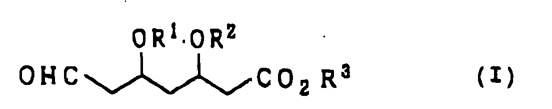
wherein R¹ and R² are independently a hydrogen atom or a protective group for hydroxyl group, or R¹ and R² taken together constitute a protective group for hydroxyl groups; and R³ is a hydrogen atom, an alkyl group which may be substituted, or an aromatic hydrocarbon group.

### DESCRIPTION OP THE PREFERRED BMBODIMENTS

The heptanoate derivative of the present invention is the heptanoate derivative of the following general formula (I): 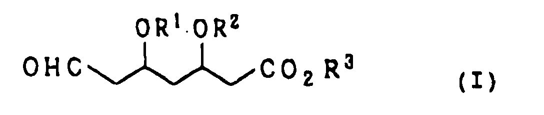
wherein R¹ and R² are independently a hydrogen atom or a protective group for hydroxyl group, or such groups that may integrally form a ring through the oxygen atoms respectively bonded thereto, which constitutes a protective group for hydroxyl groups; and R³ is a hydrogen atom, an alkyl group which may be substituted, or an aromatic hydrocarbon group.

The above heptanoate derivative of formula (I) can be produced by subjecting the octenoic acid derivative of the following formula (II) to ozonolysis: 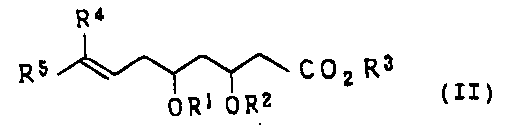
wherein R¹, R², and R³ are respectively the same as defined in the above formula (I), and R⁴ and R⁵ are independently a hydrogen atom, an alkyl group, an aromatic hydrocarbon group or a heterocyclic group, and R⁴ and R⁵ may taken together form a cycloalkylidene ring.

The above octenoic acid derivative of formula (II) can be easily produced from a butenoic acid derivative having R⁴ and R⁵ in accordance with the following reaction scheme: 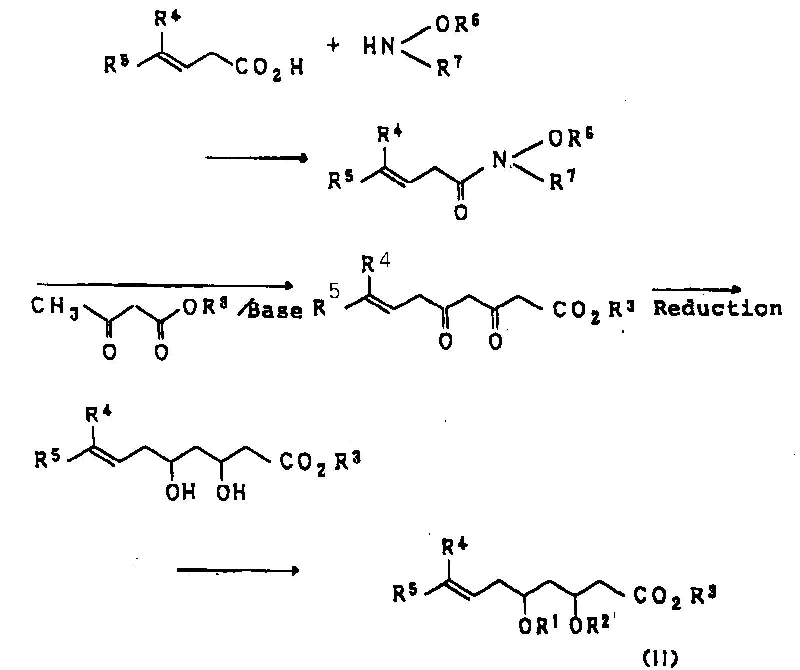
wherein R¹, R², R³, R⁴ and R⁵ are respectively the same as defined in the above formula (I) and formula (II), and R⁶ and R⁷ are an alkyl group.

The above-mentioned ozonolysis can be carried out by allowing the octenoic acid derivative of formula (II) to react with ozone to produce peroxide of the octenoic acid derivative and reducing the peroxide.

Examples of the reducing agent for use in the above ozonolysis include triphenylphosphine, dimethylsulfide, and trialkylphosphite.

It is preferable that the above reaction be carried out in an inert solvent. Examples of such an inert solvent include a halogenated hydrocarbon such as dichloromethane, 1,2-dichloroethane, and chloroform; and an alcohol such as methanol and ethanol. This reaction can usually be carried out at -78°C to room temperature.

In addition to the above-mentioned method of producing the heptanoate derivative of formula (I) by allowing the octenoic acid derivative of formula (II), the heptanoate derivative of formula (I) can also be produced by the epoxidation of the octenoic acid derivative of formula (II), followed by the treatment with periodate, or by the oxidation using an oxidizing agent such as osmium oxide.

As mentioned previously, in the heptanoate derivative of formula (I), R¹ and R² are independently a hydrogen atom or a protective group for hydroxyl group, or R¹ and R² taken together constitute a protective group for hydroxyl groups; and R³ is a hydrogen atom, an alkyl group which may be substituted, or an aromatic hydrocarbon group.

Examples of the protective group for hydroxyl group represented by R¹ or R² include an alkyl group, an alkenyl group, a heterocyclic group, a silyl group having a substituent, an acyl group, an oxycarbonyl group having a substituent, and a carbamoyl group having a substituent.

Examples of the protective group formed integrally by R¹ and R² include a methylene group, an alkylidene group optionally substituted and a carbonyl group.

A specific example of the alkyl group serving as a protective group for hydroxyl group represented by R¹ or R² is an alkyl group having 1 to 6 carbon atoms, which may have a substituent.

Examples of the substituent of the alkyl group represented by R¹ or R² are an alkoxyl group having 1 to 6 carbon atoms, a phenyl group and a naphthyl group.

Specific examples of the alkyl group optionally substituted , serving as a protective group for hydroxyl group, represented by R¹ or R² are as follows: a methyl group, a methoxymethyl group, an ethoxymethyl group, a methoxyethyl group, a methylthiomethyl group, a benzyloxymethyl group, a t-butoxymethyl group, a 2-methoxyethoxymethyl group, a 2,2, 2-trichloroethoxymethyl group, a di(2-chloroethoxy)methyl group, a 1-ethoxyethyl grouop, a 1-methyl-1-methoxyethyl group, a 1-(isopropoxy)ethyl group, a 2,2,2-trichloroethyl group, a t-butyl group, a benzyl group, a p-methoxybenzyl group, an o-nitrobenzyl group, a p-nitrobenzyl group, a p-chlorobenzyl group, an o-chlorobenzyl group, p-cyanobenzyl group, a diphenylmethyl group, an α-naphthyldiphenylmethyl group, a triphenylmethyl group, a di(p-methoxyphenyl)methyl group, a p-methoxybenzyloxymethyl group, a methoxyphenyloxymethyl group, a (4-penten-1-yl)oxymethyl group, a 2-(trimethylsilyl)ethoxymethyl group, a 2,6-dichlorobenzyl group, and a p,p'-dinitrobenzhydryl group.

An example of the alkenyl group serving as a protective group for hydroxyl group, represented by R¹ or R² is an alkenyl group having 2 to 6 carbon atoms which may be substituted, for example, by a phenyl. Specific examples of the alkenyl group include allyl group, methallyl group, and cinnamyl group.

Examples of the heterocyclic group represented by R¹ or R² are 5-membered or 6-membered saturated heterocyclic groups. Specific examples of such heterocyclic groups are a 2-tetrahydropyranyl group, a 2-tetrahydrothiopyranyl group, a 4-methoxytetrahydropyran-4-yl group, a 4-methoxytetrahydrothiopyran-4-yl group, a 2-tetrahydrofuranyl group, a 2-tetrahydrothiofuranyl group, a 3-bromotetrahydropyran-2-yl group, and a 1,4-dioxan-2-yl group.

Examples of the substituent of the silyl group represented by R¹ or R² are an alkyl group having 1 to 6 carbon atoms, and a phenyl group.

Specific examples of the silyl group having such a substitutent, represented by R¹ or R², are a trimethylsilyl group, a triethylsilyl group, an isopropyldimethylsilyl group, a t-butyldimethylsilyl group, a t-butyldiphenylsilyl group, a methyldiisopropylsilyl group, a methyldi-t-butylsilyl group, a tribenzylsilyl group, a triphenylsilyl group, and a triisopropylsilyl group.

A preferable example of the acyl group represented by R¹ or R² is an acyl group having 1 to 6 carbon atoms, which may have a substituent, such as a halogen atom, an alkoxyl group having 1 to 6 carbon atoms, a phenoxy group and a phenyl group.

Specific examples of the acyl group having such a subsitutent, represented by R¹ or R², are a formyl group, an acetyl group, a propionyl group, a chloroacetyl group, a dichloroacetyl group, a trichloroacetyl group, a trifluoroacetyl group, a methoxyacetyl group, a triphenylmethoxyacetyl group, a phenoxyacetyl group, a p-chlorophenoxyacetyl group, a 2,6-dichloro-4-methylphenoxyacetyl group, a phenylacetyl group, a chlorodiphenylacetyl group, a 3-phenylpropionyl group, a 3-benzoylpropionyl group, an isobutyroyl group, a monosuccinoyl group, a 4-oxopentanoyl gorup, a pivaloyl group, a 2-butenoyl group, an (E)-2-methyl-2-butenoyl group, a benzoyl group, a 2-chlorobenzoyl group, a 3-nitrobenzoyl group, a 2-fluorobenzoyl group, a 3-trifluoromethylbenzoyl group, a 3-trichloromethylbenzoyl group, a 4-phenylbenzoyl group, a 2,4,6-trimethylbenzoyl group, and an α-naphthoyl group.

Preferable examples of the substituent of the oxycarbonyl group represented by R¹ or R² are an alkyl group having 1 to 6 carbon atoms, an alkenyl group having 2 to 6 carbons, and a phenyl group.

Specific examples of the oxycarbonyl group having such a subsitutent, represented by R¹ or R², are a methoxycarbonyl group, an ethoxycarbonyl group, a 2,2,2-trichloroethoxycarbonyl group, an isobutoxycarbonyl group, a vinyloxycarbonyl group, an allyloxycarbonyl group, a cinnamyloxycarbonyl group, a p-nitrophenoxycarbonyl group, a benzyloxycarbonyl group, a p-methoxybenzyloxycarbonyl group, a 3,4-dimethoxybenzyloxycarbonyl group, an o-nitrobenzyloxycarbonyl group, a p-nitrobenzyloxycarbonyl group, a fluorenylmethyloxycarbonyl group, and a 2-(trimethylsilylethyl)oxycarbonyl group.

Preferable examples of the substituent of the carbamoyl group represented by R¹ or R² are an alkyl group having 1 to 6 carbon atoms, a phenyl group, and a naphthyl group.

Specific examples of the carbamoyl group having such a substituent, represented by R¹ or R² are a phenylcarbamoyl group, a naphthylcarbamoyl group, a toluylcarbamoyl group, a fluorophenylcarbamoyl group, a difluorophenylcarbamoyl group, a nitrophenylcarbamoyl group, a cyanophenylcarbamoyl group, a benzylcarbamoyl group, a methylcarbamoyl group, an ethylcarbamoyl group, an isopropylcarbamoyl group, a butylcarbamoyl group, a cyclohexylcarbamoyl group, a cyclopropylmethylcarbamoyl group, a phenylthiocarbamoyl group, a naphthylthiocarbamoyl group, a toluylthiocarbamoyl group, a fluorophenylthiocarbamoyl group, a difluorophenylthiocarbamoyl group, a nitrophenylthiocarbamoyl group, a cyanophenylthiocarbamoyl, a benzylthiocarbamoyl, a propylthiocarbamoyl group, and a butylthiocarbamoyl group.

Furthermore, R¹ and R² may integrally constitute a protective layer for hydroxyl groups. An example of the protective group is an alkylidene group having 2 to 10 carbon atoms, which may have a substituent such as a halogen atom, an alkoxyl group having 1 to 6 carbon atoms, an amino group, and a phenyl group.

Specific examples of the alkylidene group having 2 to 10 carbon atoms, which is integrally formed by R¹ and R² are an ethylidene group, a 1-t-butylethylidene group, a 1-phenylethylidene group, a 2,2,2-trichloroethylidene group, an isopropylidene group, a butylidene group, a cyclopentylidene group, a cyclohexylidene group, a cycloheptylidene group, a benzylidene group, a p-methoxybenzylidene group, a 2,4-dimethoxybenzylidene group, a p-dimethylaminobenzylidene group, an o-nitrobenzylidene group, a methoxyethylene group, an ethoxymethylene group, a dimethoxymethylene group, a 1-methoxyethylidene group, a 1,2-dimethoxyethylidene group, an α-methoxybenzylidene group, a 1-(N,N-dimethylamino)ethylidene group, and a 2-oxacyclopentylidene group.

R³ is a hydrogen atom, an alkyl group, or an aromatic hydrocarbon group.

A preferable example of the alkyl group represented by R³ is an alkyl group having 1 to 12 carbon atoms, which may be substituted by, for example, a phenyl group and a naphthyl group.

Specific examples of the alkyl group, which may be substituted , represented by R³ are a methyl group, an ethyl group, a propyl group, an isopropyl group, a n-butyl group, an isobutyl group, a sec-butyl group, a t-butyl group, a n-pentyl group, an isopentyl group, a n-hexyl group, an octyl group, a nonyl group, a decyl group, an undecyl group, and a dodecyl group.

Preferable examples of the aromatic group represented by R³ are a phenyl group and a naphthyl group.

Specific examples of the heptanoate derivative of formula (I) are as follows:
Methyl 3,5-dimethoxymethoxy-7-oxoheptanoate,
Methyl 3,5-diethoxymethoxy-7-oxoheptanoate,
Methyl 3,5-di(2-methoxyethoxy)-7-oxoheptanoate,
Methyl 3,5-dibenzyloxy-7-oxoheptanoate,
Methyl 7-oxo-3,5-di(2-propenyloxy)heptanoate,
Methyl 3,5-di(2-methyl-2-propenyloxy)-7-oxoheptanoate,
Methyl 7-oxo-3,5-di(tetrahydropyran-2-yloxy)heptanoate,
Methyl 3,5-di(trimethylsilyloxy)-7-oxoheptanoate,
Methyl 3,5-di(t-butyldimethylsilyloxy)-7-oxoheptanoate,
Methyl 3,5-di(dimethylphenylsilyloxy)-7-oxoheptanoate,
Methyl 3,5-diformyloxy-7-oxoheptanoate,
Methyl 3,5-diacetoxy-7-oxoheptanoate,
Methyl 3,5-dibenzoyloxy-7-oxoheptanoate,
Methyl 3,5-di(t-butoxycarbonyloxy)-7-oxoheptanoate,
Methyl 3,5-di(methoxycarbonyloxy)-7-oxoheptanoate,
Methyl 3,5-di(benzyloxycarbonyloxy)-7-oxoheptanoate,
Methyl 3,5-di(N-phenylcarbamoyloxy)-7-oxoheptanoate,
Methyl 3,5-O-methylidene-7-oxo-3,5-dihydroxyheptanoate,
Methyl 3,5-O-ethylidene-7-oxo-3,5-dihydroxyheptanoate,
Methyl 3,5-O-isopropylidene-7-oxo-3,5-dihydroxyheptanoate,
Methyl 3,5-O-cyclohexylidene-7-oxo-3,5-dihydroxyheptanoate,
Methyl 3,5-O-benzylidene-7-oxo-3,5-dihydroxyheptanoate,
Methyl 3,5-O-cyclopentylidene-7-oxo-3,5-dihydroxyheptanoate,
Methyl 3,5-O-carbonyl-7-oxo-3,5-dihydraxyheptanoate,
Ethyl 3,5-O-isopropylidene-7-oxo-3,5-dihydroxyheptanoate,
Propyl 3,5-O-isopropylidene-7-oxo-3,5-dihydroxyheptanoate,
t-butyl 3,5-O-isopropylidene-7-oxo-3,5-dihydroxyheptanoate, and
Phenyl 3,5-O-isopropylidene-7-oxo-3,5-dihydroxyhoptanoate

The features of the present invention will become apparent in the course of the following description of exemplary embodiments, which are given for illustration of the invention and are not intended to be limiting thereof.

### Reference Example 1

### (E) N-Methoxy-N-methyl-4-phenyl-3-butenamide:

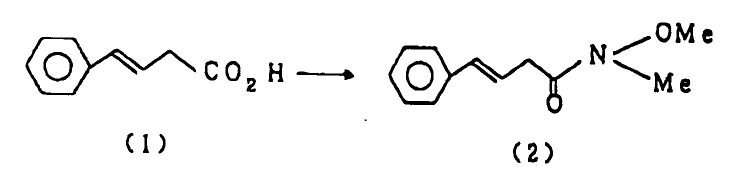

In a stream of nitrogen, 2.70 ml (37.0 mmol) of thionly chloride and one drop of N,N-dimethylformamide (DMF) were added to a solution of 3.00 g (18.5 mmol) of 4-phenyl-3-butenoic acid in 15 ml of dichloromethane at room temperature, and this reaction mixture was refluxed for 50 minutes.

From this reaction mixture, dichloromethane and excessive thionyl chloride were distilled, and 50 ml of 1,2-dichloroethane, 2.00 g (20.5 mmol) of N,O-dimethylhydroxylamine hydrochloride and 3.50 ml (43.3 mmol) of pyridine were successively added to this reaction mixture in a stream of nitrogen at room temperature. This reaction mixture was then stirred for 1.5 hours.

The reaction mixture was then added to a saturated aqueous solution of sodium chloride and the mixture was extracted with ethyl acetate.

The ethyl acetate extract layer was successively washed with 1N hydrochloric acid, a saturated aqueous solution of sodium chloride, an aqueous solution of sodium hydrogencarbonate and a saturated aqueous solution of sodium chloride, dried over magnesium sulfate, and then concentrated.

The residue was chromatographed on silica gel and eluted with a mixed solvent of hexane and ethyl acetate (1 : 1), whereby (E)-N-methoxy-N-methyl-4-phenyl-3-butenamide (compound (2)) was obtained in the form of a red oil in a yield of 3.32 g (87.5 %).
¹HNMR (300HHz, CDCl₃)
δ 3.21 (s, 3H), 3.39 (d, J=6.7Hz, 2H), 3.73 (s, 3H), 6.37 (dt, J=15.9 and 6.7Hz, 1H), 6.52 (d, 15.9Hz, 1H), 7.18 - 7.42 (m, 5H)ppm.
IR (liquid film): 2972, 2940, 1668cm⁻¹.
Mass (m/z, %): 205 (M⁺, 32), 149 (55), 144 (35), 117 (100), 115 (38), 91 (25), 69 (24).

### Reference Example 2

### Ethyl (E)-3,5-dioxo-8-phenyl-7-octenoate:

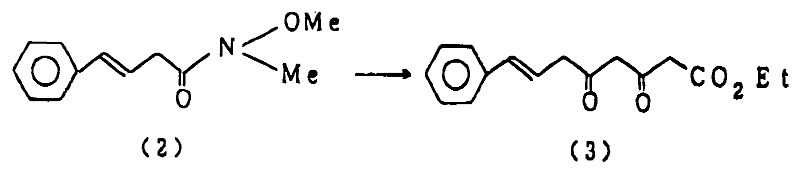

In a stream of argon, 3.21 g (80.3 mmol) of a 60% sodium hydride was suspended in 80 ml of anhydrous tetrahydrofuran (THF), and 10.8 ml (84.7 mmol) of ethyl acetoacetate was added to this suspension at 0°C, and the mixture was stirred at room temperature for 30 minutes.

This solution was cooled to 0°C, and 52.0 ml of butyllithium (1.66 M hexane solution) was added thereto. The mixture was stirred at room temperature for 20 minutes and was then cooled to -78°C, and a solution of 10.9 g (52.9 mmol) of (E)-N-methoxy-N-methyl-4-phenyl-3-butenamide (Compound (2)) synthesized in Reference Example 1 in 50 ml of anhydrous THF was added dropwise over a period of 10 minutes, and the mixture was stirred for 1 hour.

This reaction mixture was then poured into diluted hydrochloric acid. The mixture was extracted with ethyl acetate.

The ethyl acetate extract layer was washed with a saturated aqueous solution of sodium chloride, dried over magnesium sulfate, and then concentrated.

The residue was chromatographed on silica gel and eluted with a mixed solvent of hexane and ethyl acetate (3 : 1), whereby ethyl (E)-3,5-dioxo-8-phenyl-7-octenoate (Compound (3)) was obtained in the form of a colorless oil in yield of 9.76 g (67.3 %).
¹HNMR (300MHz, CDCl₃) Keto:enol 12:88 mixture
δ 1.27 (t, J=7.1Hz, 3H), 3.24 (dd, J=7.1 and 1.2Hz, 1.76H), 3.34 (s, 1.76H), 3.41 - 3.46 (m, 0.24H), 3.56 (s, 0.24H), 3.81 (s, 0.24H), 4.18 (q, J=7.1Hz, 0.24H), 4.19 (q, J=7.1Hz, 1.76H), 5.67 (s, 0.88H), 6.24 (dt, J=15.8 and 7.2Hz, 0.88H), 6.18 - 6.32 (m, 0.12H), 6.52 (d, J=15.8Hz, 1H), 7.20 - 7.42 (m, 5H)ppm.
IR (liquid film): 2988, 1740, 1600cm⁻¹.
Mass (m/z, %): 274 (M⁺, 10), 187 (12), 157 (100), 144 (26), 117 (34), 115 (80), 91 (13).

### Reference Example 3

### Ethyl (E)-3, 5-dihydroxy-8-phenyl-7-octenoate:

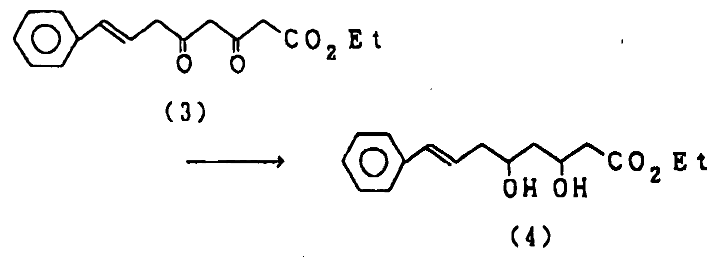

In a stream of 9.48 g (34.6 mmol) of Compound (3) synthesized in Reference Example 2 was added to a mixed solvent of 50 ml of anhydrous THF and 18 ml of methanol To this solution, 40.0 ml (40.0 mmol) of diethylmethoxyborane (1.0 M THF solution) was added at -78°C by use of a cooling bath. The cooling bath was removed and the reaction mixture was then stirred at room temperature for 30 minutes. The reaction mixture was again cooled to -78°C, and 2.67 g (70.6 mmol) of sodium borohydride was added in three portions to the reaction mixture. The mixture was stirred for 2 hours and 15 minutes.

The reaction mixture was gradually added to 400 ml of a 30% aqueous solution of hydrogen peroxide at 0°C. This mixture was then stirred at room temperature overnight.

This reaction mixture was then poured into a saturated aqueous solution of sodium chloride. The mixture was extracted with ethyl acetate.

The ethyl acetate extract layer was successively washed with a saturated aqueous solution of sodium chloride, an aqueous solution of sodium thiosulfate, and a saturated aqueous solution of sodium chloride, dried over magnesium sulfate, and then concentrated.

The residue was chromatographed on silica gel and eluted with a mixed solvent of hexane and ethyl acetate (1 : 1), whereby ethyl (E)-3,5-dihydroxy-8-phenyl-7-octenoate (Compound (4)) was obtained in a yield of 7.56 g (79.2 %).
Melting point: 44.0 - 45.0°C (colorless particles, recrystallized from a mixed solvent of ethyl acetate and hexane)
¹HNMR (300MHz, CDCl₃)
δ 1.27 (t, J=7.2Hz, 3H), 1.56 - 1.72 (m, 2H), 2.41 (t with fine coupling, J=6.7Hz, 2H), 2.38 - 2.56 (m, 2H), 3.34 (d, J=1.9Hz, 1H), 3.78 (d, J=2.4Hz, 1H), 3.97 - 4.07 (m, 1H), 4.17 (q, J=7.2Hz, 2H), 4.24 -4.35 (m, 1H), 6.23 (dt, J=15.8 and 7.3Hz, 1H), 6.47 (d, J=15.8Hz, 1H), 7.18 - 7.39 (m, 5H) ppm.
IR (KBr): 3456, 2952, 1724, 1598cm⁻¹.
Mass (FAB-positive, m/z, %): 279 ([M+H]⁺, 100)
Mass (m/z, %): 242 (M-36, 37), 143 (59), 118 (35), 117 (76), 115 (73), 97 (100), 91 (33), 73 (37).

### Reference Example 4

### Ethyl (E)-8-phenyl-3,5-O-isopropyliden-3,5-dihydroxy-7-octenoate

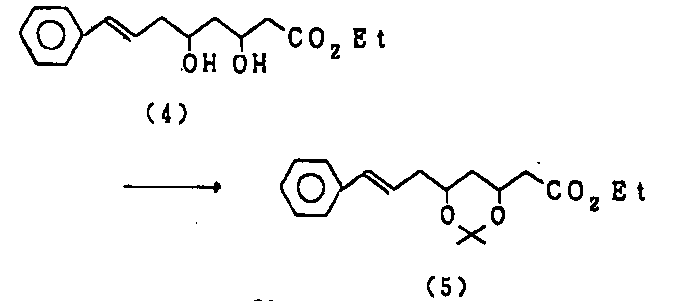

In a stream of argon, 2.00 g (7.19 mmol) of Compound (4) synthesized in Reference Example 3 was dissolved in 7.0 ml of 2,2-dimethoxypropane at room temperature. To this solution, 18 mg (0.09 mmol) of p-toluenesulfonic acid monohydrate was added, and the mixture was stirred for 2 hours.

This reaction mixture was then poured into an aqueous solution of sodium hydrogencarbonate. This mixture was extracted with ethyl acetate.

The ethyl acetate extract layer was washed with a saturated aqueous solution of sodium chloride, dried over magnesium sulfate, and then concentrated.

The residue was chromatographed on silica gel and eluted with a mixed solvent of hexane and ethyl acetate (3 : 1), whereby ethyl (E)-8-phenyl-3,5-O-isopropylidene-3,5-dihydroxy-7-octonoate (Compound (5)) was obtained in the form of a colorless oil in a yield of 2.18 g (95.4 %).
¹HNMR (300MHz, CDCl₃)
δ 1.25 (t, J=7.1Hz, 3H), 1.17 - 1.31 (m, 1H), 1.40 (s, 3H), 1.47 (s, 3H), 1.64 (dt, J=12.8 and 2.5Hz, 1H), 2.25 - 2.53 (m, 2H), 2.37 (dd, J=15.4 and 6.1Hz,1H), 2.53 (dd, J=15.4 and 7.0Hz, 1H), 3.98 (dtd, J=11.4, 6.2 and 2.5Hz, 1H), 4.06 - 4.22 (m, 2H), 4.26 - 4.37 (m, 1H), 6.20 (dt, J=15.9 and 7.1Hz, 1H), 6.44 (d, J=15.9Hz, 1H), 7.16 - 7.40 (m, 5H)ppm.
IR (liquid film): 2992, 2944, 1740, 1600cm⁻¹.
Mass (FAB-positive, m/z, %): 319 ([M+H]⁺,6).
Mass (m/z, %): 303 (M-15, 26), 201 (75), 183 (34), 155 (39), 143 (100), 129 (38), 115 (49), 97 (56).

### Example 1

### Ethyl 7-oxo-3,5-O-isopropyliden-3,5-dihydroxyheptanoate:

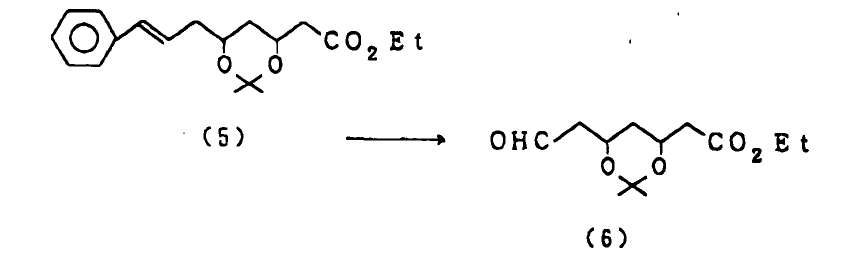

1.50 g (4.72 mmol) of Compound (5) synthesized in Reference Example 4 was added to 100 ml of dichloromethane. Ozone was passed through this solution at -78°C for 1 hour. Excessive ozone was then removed from this reaction mixture by purging it with argon until the bluish reaction mixture became colorless.

To this reaction mixture, 1.50 g (5.72 mmol) of triphenylphosphine was added, and the reaction mixture was stirred at -78°C for 30 minutes, and then at room temperature for 1 hour.

This reaction mixture was concentrated, and the residue was chromatographed on silica gel and eluted with a mixed solvent of hexane and ethyl acetate (2 : 1), whereby ethyl 7-oxo-3,5-O-isopropylidene-3,5-dihydroxyheptanoate (Compound (6)) was obtained in the form of a colorless oil in a yield of 1.10 g (95.4 %).
¹HNMR (300MHz, CDCl₃)
δ 1.27 (t, J=7.1Hz, 3H), 1.22 - 1.35 (m, 1H), 1.37 (s, 3H), 1.50 (s, 3H), 1.69 (dt, J=12.7 and 2.5Hz, 1H), 2.39 (dd, J=15.5 and 6.1Hz, 1H), 2.49 (ddd, J=16.6, 5.0 and 1.6Hz, 1H), 2.55 (dd, J=15.5 and 7.1Hz, 1H), 2.63 (ddd, J=16.6, 7.3 and 2.3Hz, 1H), 4.08 - 4.25 (m, 2H), 4.32 - 4.51 (m, 2H), 9.79 (dd, J=2.3 and 1.6Hz, 1H)ppm.
IR (liquid film): 2996, 1734cm⁻¹.
Mass (FAB-positive, m/z, %): 245 ([M+H]⁺, 82).
Mass (m/z, %): 229 (M⁺-15, 100), 201 (19).

### Reference Example 5

### Ethyl 7-[4'-fluoro-5,6-dimethoxy-3-(propan-2-yl)biphenyl-2-yl]-3,5-O-isopropyliden-3,5,7-trihydroxyheptanoate:

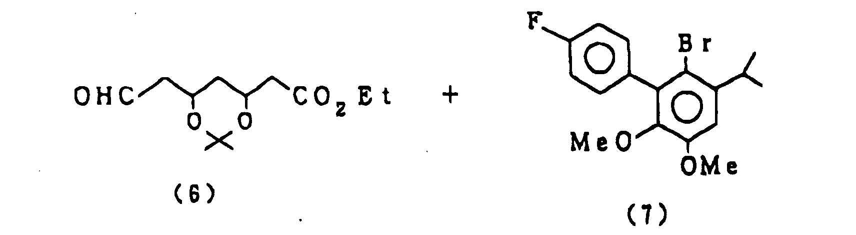

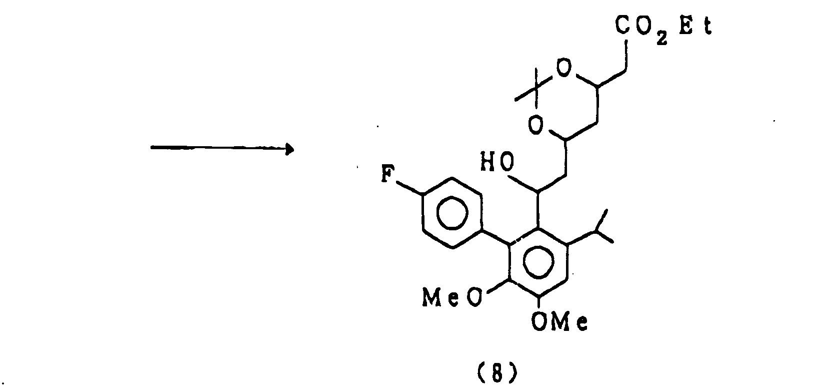

In an atmosphere of argon, 750 mg (2.11 mmol) of 2-bromo-4'-fluoro-5,6-dimethoxyl3-(propan-2-yl)biphenyl (Compound (7)) and 207 mg (8.52 mmol) of magnesium turnings were added to 4 ml of anhydrous THF, and the mixture was refluxed. To this mixture, a solution of 0.27 ml (2.10 mmol) of 1,2-diboromoethane in 4 ml of anhydrous THF was added dropwise over a period of 10 minutes, and the mixture was refluxed for 1 hour.

To this solution, a solution of 620 mg (2.54 mmol) of Compound (6) synthesized in Example 1 in 2 ml of anhydrous THF was added, and the mixture was stirred at room temperature for 1 hour.

This reaction mixture was poured into a saturated aqueous solution of ammonium chloride. The mixture was then extracted with ethyl acetate. The ethyl acetate extract layer was washed with a saturated aqueous solution of sodium chloride, dried over magnesium sulfate, and concentrated.

The residue was chromatographed on silica gel and eluted with a mixed solvent of hexane and ethyl acetate (2 : 1), whereby ethyl 7-[4'-fluoro-5,6-dimethoxy-3-(propan-2-yl)-biphenyl-2-yl]-3,5-O-isopropylidene-3,5,7- trihydroxyheptanoate (Compound (8)) was obtained in the form of a colorless amorphous solid in a yield of 694 mg (63.3%).
¹HNMR (300MHz, CDCl₃) Mixture of 1:1 stereoisomers
δ 0.97 (dd, J=24.3 and 11.8Hz, 0.5H), 1.10 (dd, J=24.3 and 11.8Hz, 0.5H), 1.22 - 1.31 (m, 15H), 1.37 - 1.44 (m, 0.5H), 1.48 - 1.60 (m, 0.5H), 2.04 - 2.54 (m, 4H), 2.87 (broad s, 0.5H), 3.46 (s, 1.5H), 3.47 (s, 1.5H), 3.75 - 3.92 (m, 1H), 3.89 (s, 3H), 3.98 - 4.28 (m, 4H), 4.68 - 4.75 (m, 0.5H), 4.87 - 4.94 (m, 0.5H), 6.91 (s, 1H), 7.00 - 7.30 (m, 4H)ppm.
IR (KBr): 3532, 2992, 2956, 1738, 1590cm⁻¹.
Mass (m/z, %): 518 (M⁺, 20), 442 (100), 424 (85), 327 (91), 285 (77), 283 (81), 183 (20), 43 (37).

### Reference Example 6

### Ethyl (E)-7-[4'-fluoro-5,6-dimethoxy-3-(propan-2-yl)biphenyl-2-yl]-3,5-O-isopropylidene-3,5-dihydroxy-6-heptenoate:

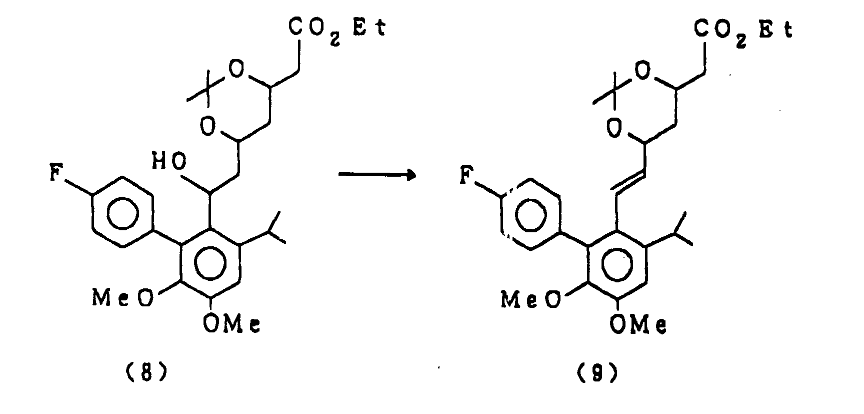

225 mg (0.43 mmol) of Compound (8) synthesized in Reference Example 5 was dissolved in 4 ml of 1,2-dichloroethane. To this solution, 0.11 ml (1.30 mmol) of anhydroud pyridine and then 0.04 ml (0.49 mmol) of thionyl chloride were added, and the mixture was stirred in an atmosphere of argon for 50 minutes.

This reaction mixture was poured into water, and the mixture was then extracted with ethyl acetate.

The ethyl acetate extract layer was washed with an aqueous solution of sodium hydrogencarbonate and a saturated aqueous solution of sodium chloride, dried over magnesium sulfate, and then concentrated.

The residue was dissolved in 5 ml of 1,2-dichloroethane, and to this solution, 0.10 ml (1.24 mmol) of anhydrous pyridine was added, and the mixture was refluxed in an atmosphere of argon for 5 hours.

This reaction mixture was poured into water, and the mixture was extracted with ethyl acetate. The ethyl acetate extract layer was successively washed with a saturated aqueous solution of sodium chloride, an aqueous solution of sodium hydrogencarbonate, and a saturated aqueous solution of sodium chloride, dried over magnesium sulfate, and concentrated.

The residue was crystallized from a mixed solvent of ethyl acetate and hexane, whereby ethyl (E)-7-[4'-fluoro-5,6-dimethoxy-3-(propan-2-yl)-biphenyl-2-yl]-3,5-O-isopropylidene-3,5-dihydroxy-6-heptenoate (compound (9)) was obtained in a yield of 129 mg (59.4%).

The filtrate was concentrated and the residue was chromatographed on silica gel and eluted with a mixed solvent of ethyl acetate and hexane (1 : 4), whereby Compound (9) was obtained in a yield of 42 mg (19.4%).
Melting point: 120.0 - 121.0°C (colorless needles, recrystallized from a mixed solvent of ethyl acetate and hexane)
¹HNMR (300MHz, CDCl₃)
δ 0.88 (dd, J=24.5 and 11.7Hz, 1H), 1.20 - 1.30 (m, 1H), 1.23 (d, J=6.8Hz, 6H), 1.27 (t, J=7.2Hz, 3H), 1.34 (s, 3H), 1.43 (s, 3H), 2.30 (dd, J=15.5 and 6.2Hz, 1H), 2.49 (dd, J=15.5 and 7.0Hz, 1H), 3.25 (hept, J=6.8Hz, 1H), 3.47 (s, 3H), 3.91 (s, 3H), 4.09 - 4.30 (m, 4H), 5.13 (dd, J=16.2 and 6.2Hz, 1H), 6.22 (dd, J=16.2 and 0.9Hz, 1H), 6.85 (s, 1H), 6.98 - 7.07 (m, 2H), 7.10 - 7.20 (m, 1H)ppm.
IR (KBr): 2964, 2944, 1738, 1602cm⁻¹.
Mass (m/z, %): 500 (M⁺, 13), 442 (71), 424 (100), 381 (36), 327 (74), 285 (76), 283 (95), 183 (24), 43 (45).

### Reference Example 7

### Ethyl (E)-7-[4'-fluoro-5,6-dimethoxy-3-(propan-2-yl)biphenyl-2-yl]-3,5-dihydroxy-6-heptenoate:

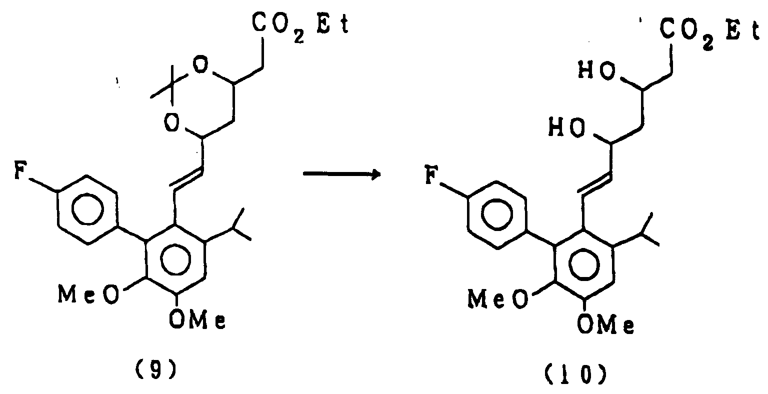

51 mg (0.10 mmol) of Compound (9) synthesized in Reference Example 6 was dissolved in a mixed solvent of 2 ml of THF and 1 ml of water. To this solution, 18 mg (0.10 mmol) of p-toluenesulfonic acid monohydrate was added, and the mixture was stirred in an atmosphere of argon at room temperature for 4 days.

The reaction mixture was poured into water, and the mixture was then extracted with ethyl acetate. The ethyl acetate extract layer was successively washed with water, a saturated aqueous solution of sodium hydrogencarbonate, and a saturated aqueous solution of sodium chloride, dried over magnesium sulfate, and concentrated.

The residue was chromatographed on silica gel and eluted with a mixed solvent of ethyl acetate and hexane (1 : 1), whereby ethyl (E)-7-[4'-fluoro-5,6-dimethoxy-3-(propan-2-yl)-biphenyl-2-yl]-3,5-dihydroxy-6-heptenoate (compound (10)) was obtained in a yield of 34 mg (72.5 %).
Melting point: 77.0 - 78.5°C (colorless needles, recrystallized from a mixed solvent of ethyl acetate and hexane)
¹HNMR (300MHz, CDCl₃)
δ 1.22 (d, J=6.8Hz, 3H), 1.23 (d, J=6.8Hz, 3H), 1.15 - 1.30 (m, 1H), 1.29 (t, J=7.2Hz, 3H), 1.34 - 1.49 (m, 1H), 2.32 - 2.48 (m, 2H), 2.75 - 2.79 (m, 1H), 3.22 (hept, J=6.8Hz, 1H), 3.48 (s, 3H), 3.58 (s with fine coupling, 1H), 3.91 (s, 3H), 4.00 - 4.12 (m, 1H), 4.18 (q, J=7.2Hz, 2H), 4.22 - 4.34 (m, 1H), 5.16 (dd, J=16.1 and 6.5Hz, 1H), 6.34 (dd, J=16.1 and 1.1Hz, 1H), 6.86 (s, 1H), 7.00-7.09 (m, 2H), 7.10 - 7.20 (m, 2H)ppm.
IR (KBr): 3432, 1724cm⁻¹.
Mass (m/z, %): 460 (M⁺, 100), 442 (88), 327 (51), 285 (56), 273 (65), 243 (28), 183 (17).

### Reference Example 8

### Sodium (E)-7-[4'-fluoro-5,6-dimethoxy-3-(propan-2-yl)biphenyl-2-yl]-3,5-dihydroxy-6-heptenoate:

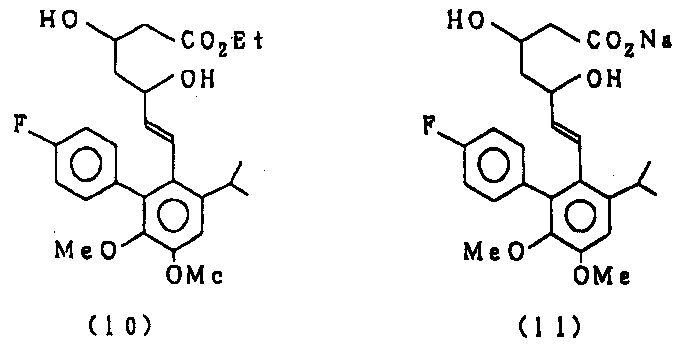

122 mg (0.265 mmol) of Compound (10) synthesized in Reference Example 7 was dissolved in 3 ml of ethanol. To this solution, 0.530 ml (0.265 mmol) of a 0.5 N aqueous solution of sodium hydroxide was added, and the mixture was stirred in an atmosphere of argon at room temperature for 1 hour and 25 minutes.

The reaction mixture was concentrated, and the residue was dissolved in water and then subjected to freeze-drying, whereby sodium (E)-7-[4'-fluoro-5,6-dimethoxy-3-(propan-2-yl)biphenyl-2-yl]-3,5-dihydroxy-6-heptenoate (compound (11)) was obtained in the form of a colorless amorphous solid in yield of 117 mg (97.2 %).
¹HNMR (300MHz, CD₃OD)
δ 1.24 (d, J=6.8Hz, 6H), 1.20 - 1.31 (m, 1H), 1.45 -1.57 (m, 1H), 2.16 (dd, J=15.4 and 7.9Hz, 1H), 2.28 (dd, J=15.4 and 4.5Hz, 1H), 3.35 (hept, J=6.8Hz, 1H), 3.45 (s, 3H), 3.71 - 3.82 (m, 1H), 3.89 (s, 3H), 4.12 - 4.22 (m, 1H), 5.21 (dd, J=16.1 and 6.7Hz, 1H), 6.29 (dd, J=16.1 and 1.1Hz, 1H), 6.95 (s, 1H), 7.04 - 7.20 (m, 4H)ppm.
IR (KBr): 3456, 2969, 1574cm⁻¹.
Mass (FAB-negative, m/z, %): 453 ([M-H]⁻, 7), 431 (100).

### Reference Example 9

### Ethyl (E)-8-phenyl-3,5-O-cyclohexylidene-3,5-dihydroxy-7-octenoate:

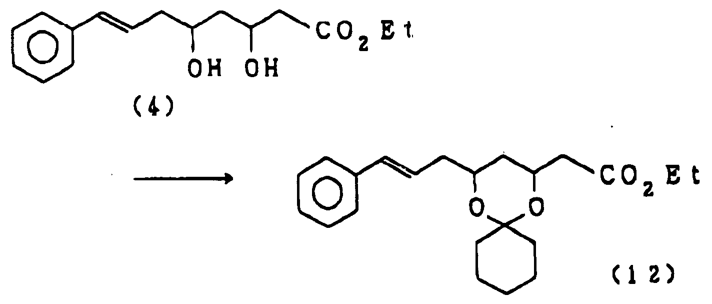

In an atmosphere of argon, 1.09 g (3.92 mmol) of Compound (4) synthesized in Reference Example 3 was dissolved in 3 ml of cyclohexanone dimethylacetal, and the mixture was stirred at room temperature. To this reaction mixture, 35 mg (0.18 mmol) of p-toluenesulfonic acid monohydrate was added, and the mixture was stirred for 4 hours and 25 minutes.

This reaction mixture was poured into water, and the mixture was then extracted with ethyl acetate. The ethyl acetate extract layer was successively washed with an aqueous solution of sodium hydrogencarbonate, water, and a saturated aqueous solution of sodium chloride, dried over magnesium sulfate, and concentrated.

The residue was chromatographed on silica gel and eluted with a mixed solvent of hexane and ethyl acetate (6 : 1). The eluant was concentrated and the residue was again chromatographed on silica gel, and then eluted with a mixed solvent of hexane and ethyl acetate (10 : 1), whereby ethyl (E)-8-phenyl-3,5-O-cyclohexylidene-3,5-dihydroxy-7-octenoate (compound (12)) was obtained in the form of a colorless oil in a yield of 1.14 g (81.2 %).
¹HNMR (300MHz, CDCl₃)
δ 1.18 - 1.32 (m, 1H), 1.26 (t, J=7.1Hz, 3H), 1.32 - 1.50 (m, 4H), 1.50 - 1.67 (m, 5H), 1.72 - 1.86 (m, 1H), 1.94 - 2.08 (m, 1H), 2.25 - 2.47 (m, 2H), 2.37 (dd, J=15.3 and 5.3Hz, 1H), 2.52 (dd, J=15.3 and 7.7Hz, 1H), 3.94 - 4.07 (m, 1H), 4.10 - 4.20 (m, 2H), 4.27 - 4.38 (m, 1H), 6.23 (ddd, J=15.9, 7.4 and 6.6Hz, 1H), 6.44 (d, J=15.9Hz, 1H), 7.17 - 7.38 (m, 5H)ppm.
IR (liquid film): 2932, 2860, 1734, 1598cm⁻¹.
Mass (m/z, %): 358 (M⁺, 14), 241 (100), 155 (64), 143 (74), 129 (38), 99 (48), 91 (31), 55 (16).

### Example 2

### Ethyl 7-oxo-3,5-O-cyclohexylidene-3,5-dihydroxyheptanoate:

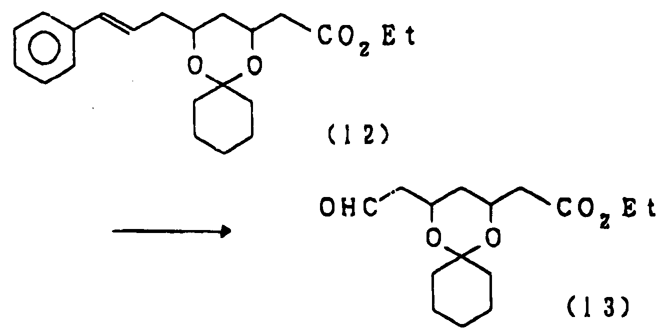

275 mg (0.77 mmol) of Compound (12) synthesized in Reference Example 9 was added to 7 ml of dichloromethane, and this solution was stirred at -78°C. Ozone was passed through this solution for 25 minutes. Excessive ozone was then purged from this reaction mixture with oxygen until the bluish reaction mixture became colorless.

To this reaction mixture, 241 mg (0.92 mmol) of triphenylphosphine was added, and the reaction mixture was stirred at room temperature for 1 hour and 35 minutes.

This reaction mixture was concentrated, and the residue was chromatographed on silica gel and eluted with a mixed solvent of hexane and ethyl acetate (2 : 1), whereby ethyl 7-oxo-3,5-O-cyclohexylidene-3,5-dihydroxyheptanoate (compound (13)) was obtained in the form of a colorless oil in a yield of 199 mg (91.2 %).
¹HNMR (300MHz, CDCl₃)
δ 1.22 - 1.36 (m, 1H), 1.27 (t, J=7.1Hz, 3H), 1.34 -1.50 (m, 4H), 1.50 - 1.63 (m, 4H), 1.66 (d with fine coupling, J=12.7Hz, 1H), 1.82 - 2.05 (m, 2H), 2.38 (dd, J=15.3 and 5.5Hz, 1H), 2.42 - 2.50 (m, 1H), 2.52 (dd, J=15.3 and 7.7Hz, 1H), 2.62 (ddd, J-16.5, 7.8 and 2.3Hz, 1H), 4.08 - 4.22 (m, 2H), 4.32 - 4.53 (m, 2H), 9.80 (s, with fine coupling, 1H)ppm.
IR (liquid film): 2940, 2864, 1740cm⁻¹.
Mass (m/z, %): 284 (M⁺, 53), 241 (100), 99 (29), 81 (53), 55 (27).

### Reference Example 10

### Ethyl 7-[4'-fluoro-5,6-dimethoxy-3-(propan-2-yl)biphenyl-2-yl]-3,5-O-cyclohexylidene-3,5,7-trihydroxyheptanoate:

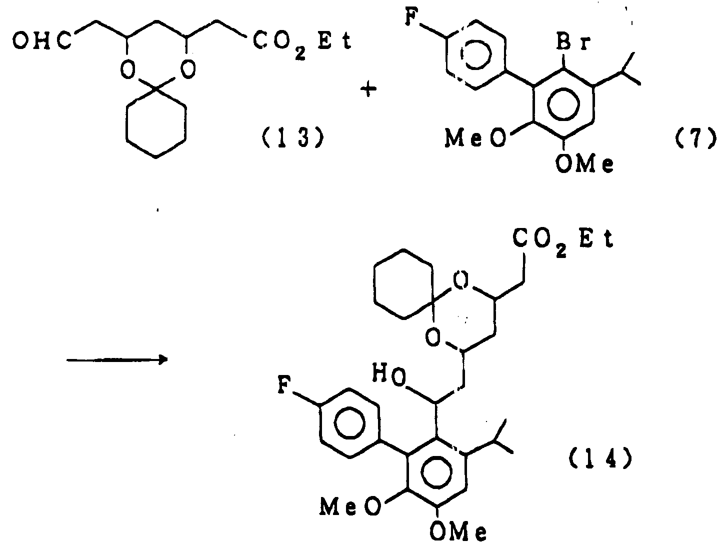

102 mg (4.90 mmol) of magnesium turnings was suspended in 1 ml of anhydrous THF. To this suspension, a solution of 0.12 ml (1.40 mmol) of 1,2-dibromoethane and 495 mg (1.40 mmol) of 2-bromo-4'-fluoro-5,6-dimethoxy-3-(propan-2-yl)biphonyl (Compound (7)) in 8 ml of anhydrous THF was added dropwise in a stream of argon over a period of 10 minutes.

This reaction mixture was refluxed for 30 minutes, and then cooled to room temperature.

To this reaction mixture, a solution of 333 mg (1.17 mmol) of Compound (13) synthesized in Reference Example 9 in 4 ml of anhydrous THF was added, and the mixture was stirred for 3 hours and 15 minutes.

This reaction mixture was then poured into 1N hydrochloric acid, and the mixture was then extracted with ethyl acetate. The ethyl acetate extract layer was successively washed with water, a saturated aqueous solution of sodium hydrogencarbonate, water, and a saturated aqueous solution of sodium chloride, dried over magnesium sulfate, and concentrated.

The residue was chromatographed on silica gel and eluted with a mixed solvent of ethyl acetate and hexane (1 : 6 to 1 : 2), whereby ethyl 7-[4'-fluoro-5,6-dimethoxy-3-(propan-2-yl)biphenyl-2-yl]-3,5-O-cyclohexylidene-3,5,7-trihydroxyheptanoate (compound (14)) was obtained in the form of a colorless amorphous solid in a yield of 242 mg (37.1%).
Mixture of 7: 3 stereoisomers
¹HNMR (300MHz, CDCl₃)
δ 0.96 - 1.09 (m, 0.7H), 1.10 - 1.22 (m, 0.3H), 1.23 - 1.39 (m, 15H), 1.39 - 1.62 (m, 4H), 1.69 - 1.90 (m, 2H), 2.07 - 2.23 (m, 1H), 2.24 - 2.37 (m, 1H), 2.40 - 2.52 (m, 1H), 3.13 - 3.16 (m, 0.3H), 3.47 (s, 3H), 3.72 - 3.98 (m, 1H), 3.99 (s, 3H), 4.03 - 4.20 (m, 3H), 4.20 - 4.32 (m, 1H), 4.70 - 4.76 (m, 0.3H), 4.93 - 5.02 (m, 0.7H), 6.91 (s, 1H), 7.00 - 7.34 (m, 4H)ppm.
IR (KBr): 3532, 2940, 2868, 1740, 1592cm⁻¹.
Mass (m/z, %): 558 (M⁺, 16), 540 (40), 442 (100), 424 (76), 327 (76), 285 (75), 283 (86), 55 (34).

### Reference Example 11

### Ethyl (E)-7-[4'-fluoro-5,6-dimethoxy-3-(propan-2-yl)biphenyl-2-yl]-3,5-O-cyclohexylidene-3,5-dihydroxy-6-heptenoate:

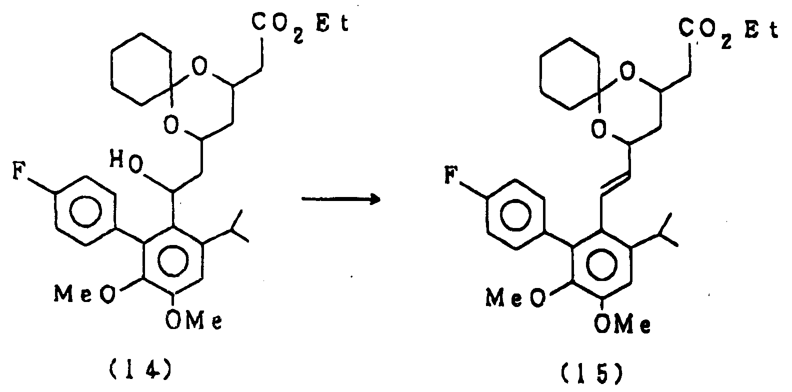

77 mg (0.14 mmol) of Compound (14) synthesized in Reference Example 10 was dissolved in 2 ml of 1,2-dichloroethane. To this solution, at 0°C, 0.03 ml (0.37 mmol) of anhydrous pyridine and then 0.01 ml (0.15 mmol) of thionyl chloride were added, and the mixture was stirred in an atmosphere of argon for 30 minutes.

This reaction mixture was poured into water, and the mixture was extracted with ethyl acetate.

The ethyl acetate extract layer was successively washed with an aqueous solution of sodium hydrogen-carbonate and a saturated aqueous solution of sodium chloride, dried over magnesium sulfate, and then concentrated.

The residue was dissolved in 3 ml of 1,2-dichloroethane, and 0.03 ml (0.37 mmol) of anhydrous pyridine was added thereto. This mixture was refluxed in an atmosphere of argon for 2 hours.

This reaction mixture was then added to water, and the mixture was then extracted with ethyl acetate. The ethyl acetate extract layer was washed with a saturated aqueous solution of sodium chloride, dried over magnesium sulfate, and concentrated.

The residue was chromatographed on silica gel and eluted with a mixed solvent of ethyl acetate and hexane (1 : 4), whereby ethyl (E)-7-[4'-fluoro-5,6-dimethoxy-3-(propan-2-yl)-biphenyl-2-yl]-3,5-O-cyclohexylidene-3,5- dihydroxy-6-heptenoate (compound (15)) was obtained in a yield of 53 mg (71.1 %).
Melting point: 118.0 - 118.5°C (colorless fine particles, recrystallized from a mixed solvent of ethyl acetate and hexane)
¹HNMR (300MHz, CDCl₃)
δ 0.97 (dd, J=24.4 and 11.7Hz, 1H), 1.22 (d, J=6.8Hz, 3H), 1.22 (d, J=6.8Hz, 3H), 1.27 (t, J=7.2Hz, 3H), 1.27 - 1.46 (m, 5H), 1.46 - 1.57 (m, 4H), 1.75 - 1.95 (m, 2H), 2.32 (dd, J=15.4 and 5.6Hz, 1H), 2.47 (dd, J=15.4 and 7.6Hz, 1H), 3.25 (hept, J=6.8Hz, 1H), 3.48 (s, 3H), 3.91 (s, 3H), 4.15 (q, J=7.2Hz, 2H), 4.20 - 4.33 (m, 2H), 5.15 (dd, J=16.2 and 6.2Hz, 1H), 6.23 (dd, J=16.2 and 1.1Hz, 1H), 6.85 (s, 1H), 6.97 - 7.05 (m, 2H), 7.10 - 7.18 (m, 2H)ppm.
IR (KBr): 2948, 2856, 1740, 1606, 1588cm⁻¹.
Mass (m/z, %): 540 (M⁺, 100), 442 (72), 399 (31), 285 (48), 283 (31), 55 (8).

### Reference Example 12

### t-Butyl (E)-3,5-dioxo-8-phenyl-7-octenoate:

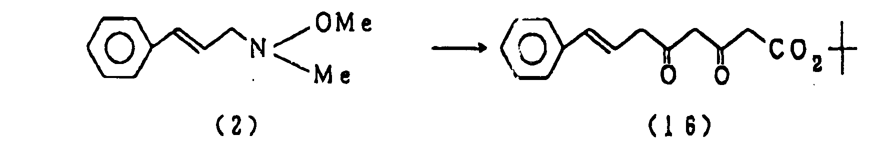

In a stream of argon, 8.10 ml (48.8 mmol) of t-butyl acetoacetate was added to a suspension of 1.78 g (44.5 mmol) of a 60% sodium hydride in 70 ml of anhydrous THF at 0°C, and the mixture was stirred at room temperature for 30 minutes.

This reaction mixture was cooled to 0°C, and 28.5 ml (47.3 mmol) of butyllithium (1.66 M hexane solution) was added, and the mixture was stirred for 20 minutes.

The reaction mixture was cooled to -78°C, and a solution of 5.88 g (28.7 mmol) of Compound (2) synthesized in Reference Example 1 in 30 ml of anhydrous THF was added to the reaction mixture over a period of 15 minutes. This mixture was stirred for 1 hour and 35 minutes.

This reaction mixture was poured into diluted hydrochloric acid, and the mixture was extracted with ethyl acetate.

The ethyl acetate extract layer was washed with a saturated aqueous solution of sodium chloride, dried over magnesium sulfate, and then concentrated.

The residue was chromatographed on silica gel and eluted with a mixed solvent of hexane and ethyl acetate (5 : 1), whereby a crude product was obtained.

This crude product was chromatographed on silica gel and eluted with a mixed solvent of dichloromethane and ethyl acetate (100 : 1), whereby t-butyl (E)-3,5-dioxo-8-phenyl-7-octenoate (compound (16)) was obtained in the form of a colorless oil in a yield of 5.05 g (58.3 %).
¹HNMR (300MHz, CDCl₃) Keto:enol 15:85 mixture,
δ 1.45 (s, 7.65H), 1.46 (s, 1.35H), 3.10 - 3.18 (m, 1.7H), 3.25 (s, 1.7H), 3.44 (dd, J=7.1 and 1.2Hz, 0.3H), 3.47 (s, 0.3H), 3.79 (s, 0.3H), 5.66 (s, 0.85H), 6.25 (dt, J=15.8 and 7.2Hz, 0.85H), 6.19 - 6.32 (m, 0.15H), 6.51 (d, J=15.8Hz, 1H), 7.20 - 7.41 (m, 5H) ppm.
IR (liquid film) : 2984, 2936, 1732, 1604 cm⁻¹.
Mass (m/z, %) : 302 (M⁺, 1), 246 (14), 229 (11), 185 (26), 129 (100), 117 (25), 57 (35).

### Reference Example 13

### t-butyl (E)-3,5-dihydroxy-8-phenyl-7-octenoate:

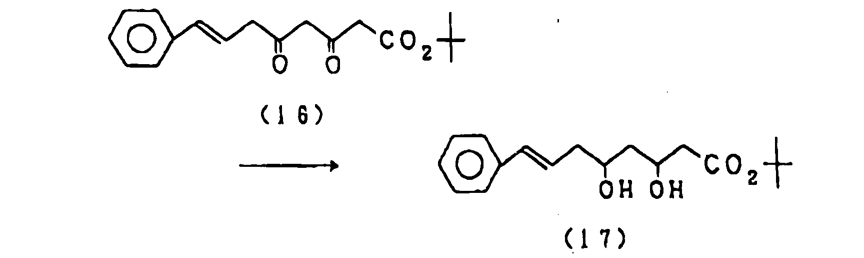

4.67 g (15,5 mmol) of Compound (16) synthesized in Reference Example 12 was dissolved in 20 ml of anhydrous THF and 10 ml of methanol. To this solution, 18.5 ml (18.5 mmol) of diethylmethoxyborane (1.0M THF solution) was added at -78°C by use of a cooling bath in a stream of argon. The cooling bath was removed and the reaction mixture was stirred at room temperature for 30 minutes.

This reaction mixture was again cooled to -78°C, and 1.20 g (31.7 mmol) of sodium borohydride was added in two portions to the reaction mixture. The mixture was stirred for 5 hours.

This reaction mixture was then gradually added, with stirring at 0°C, to 180 ml of a 30% aqueous solution of hydrogen peroxide, and the mixture was stirred at room temperature overnight.

This reaction mixture was poured into a saturated aqueous solution of sodium chloride, and the mixture was extracted with ethyl acetate.

The ethyl acetate extract layer was successively washed with a saturated aqueous solution of sodium chloride, an aqueous solution of sodium thiosulfate, and a saturated aqueous solution of sodium chloride, dried over magnesium sulfate, and then concentrated.

The residue was chromatographed on silica gel and eluted with a mixed solvent of hexane and ethyl acetate (1 : 1), whereby t-butyl (E)-3,5-dihydroxy-8-phenyl-7-octenoate (compound (17)) was obtained in the form of a colorless oil in a yield of 3.80 g (80.8 %).
¹HNMR (300MHz, CDCl₃)
δ 1.46 (s, 9H), 1.54 - 1.68 (m, 2H), 2.35 - 2.46 (m, 4H), 3.48 (s, 1H), 3.82 (d, J=2 4Hz, 1H), 3.94 - 4.08 (m, 1H), 4.18 - 4.31 (m, 1H), 6.24 (dt, J-15.9 and 7.2Hz, 1H), 6.46 (d, J=15.9Hz, 1H), 7.17 - 7.39 (m, 5H) ppm.
IR (liquid film): 3448, 2980, 2936, 1728, 1600 cm⁻¹.
Mass (FAB-positive, m/z, %) : 307 ([M+H]⁺, 5).
Mass (m/z, %) : 270 (M⁺-36, 25), 133 (45), 118 (44), 117 (42), 115 (100), 57 (60).

### Reference Example 14

### t-Butyl (E)-8-phenyl-3,5-O-isopropylidene-3,5-dihydroxy-7-octenoate:

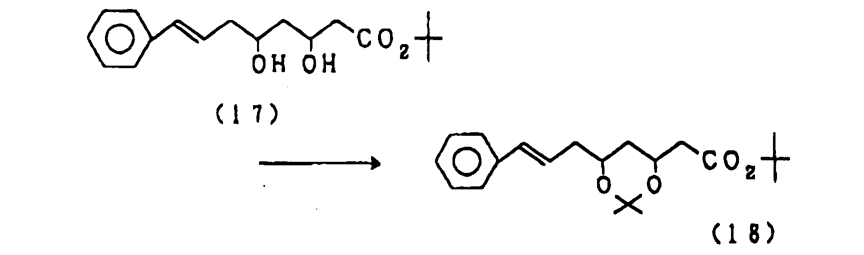

740 mg (2.43 mmol) of Compound (17) synthesized in Reference Example 13 was dissolved in 7.0 ml of 2,2-dimethoxypropane, and the mixture was stirred at room temperature in a stream of argon. To this solution, 5 mg (0.03 mmol) of p-toluonesulfonic acid monohydrate was added, and the mixture was stirred for 1 hour.

This reaction mixture was then poured into an aqueous solution of sodium hydrogencarbonate, and the mixture was then extracted with ethyl acetate. The ethyl acetate extract layer was washed with a saturated aqueous solution of sodium chloride, dried over magnesium sulfate, and concentrated.

The residue was chromatographed on silica gel and eluted with a mixed solvent of hexane and ethyl acetate (3 : 1), whereby t-butyl (E)-8-phenyl-3,5-O-isopropylidene-3,5-dihydroxy-7-octenonte (compound (18)) was obtained in the form of a colorless oil in a yield of 784 mg (93.6 %).
¹HNMR (300MHz, CDCl₃)
δ 1.16 - 1.30 (m, 1H), 1.40 (s, 3H), 1.43 (s, 9H), 1.47 (s, 3H), 1.62 (dt, J=12.8 and 2.4Hz, 1H), 2.30 (dd, J=15.0 and 6.0Hz, 1H), 2.25 - 2.52 (m, 2H), 2.43 (dd, J=15.0 and 7.2Hz, 1H), 3.97 (dtd, J=11.5, 6.2 and 2.4Hz, 1H), 4.20 - 4.32 (m, 1H), 6.14 - 6.28 (m, 1H), 6.43 (d, J=15.9Hz, 1H), 7.17 - 7.38 (m, 5H) ppm.
IR (liquid film) : 2984, 2940, 1732 cm⁻¹.
Mass (FAB-positive, m/z, %) : 347 ([M+H]⁺, 2).
Mass (m/z, %) : 331 (M-15, 9), 215 (32), 173 (100), 155 (26), 115 (57), 57 (30).

### Example 3

### t-Butyl 7-oxo-3,5-O-isopropylidene-3,5-dihydroxyheptanoate:

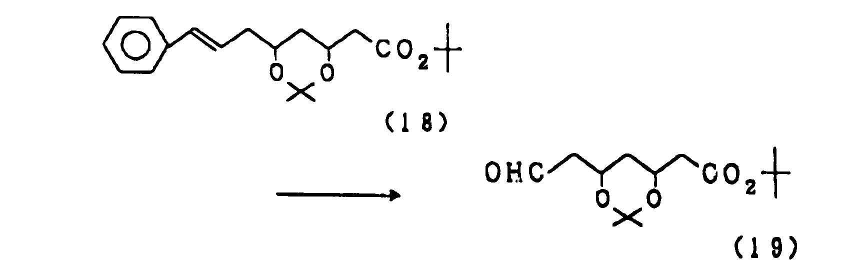

734 mg (2.13 mmol) of Compound (18) synthesized in Reference Example 14 was added to 40 ml of dichloromethane, and this solution was stirred at -78°C. Ozone was passed through this solution for 30 minutes. Excessive ozone was then purged from this reaction mixture with oxygen until the bluish reaction mixture became colorless.

To this reaction mixture, 670 mg (2.55 mmol) of triphenylphosphine was added, and the reaction mixture was stirred at for 2 hours and allowed to stand at 0°C overnight.

This reaction mixture was concentrated, and the residue was chromatographed on silica gel and eluted with a mixed solvent of hexane and ethyl acetate (2 : 1), whereby t-butyl 7-oxo-3,5-O-isopropylidene-3,5-dihydroxyheptanoate (compound (19)) was obtained in the form of a colorless oil in yield of 495 mg (85.3 %).
¹HNMR (300MHz, CDCl₃)
δ 1.20 - 1.34 (m, 1H), 1.36 (s, 3H), 1.45 (s, 9H), 1.48 (s, 3H), 1.66 (dt, J=12.7 and 2.5Hz, 1H), 2.31 (dd, J=15.1 and 6.1Hz, 1H), 2.43 (dd, J=15.1 and 7.1 Hz, 1H), 2.47 (ddd, J=16.7, 5.0 and 1.7Hz, 1H), 2.62 (ddd, J=16.7, 7.3 and 2.3Hz, 1H), 4.25 - 4.36 (m, 1H), 4.38 - 4.48 (m, 1H), 9.78 (dd, J=2.3 and 1.7Hz, 1H) ppm.
IR (liquid film) : 2988, 2944, 1726 cm⁻¹.
Mass (FAB-positive, m/z, %) : 273 ([M+H]⁺, 19).
Mass (m/z, %) : 257 (M⁺-15, 62), 201 (42), 149 (61), 141 (46), 59 (34), 57 (70).

### Reference Example 15

### t-Butyl 7-[4'-fluoro-5,6-dimethoxy-3-(propan-2-yl)biphenyl-2-yl]-3,5-O-isopropylidene-3,5,7-trihydroxyheptenoate:

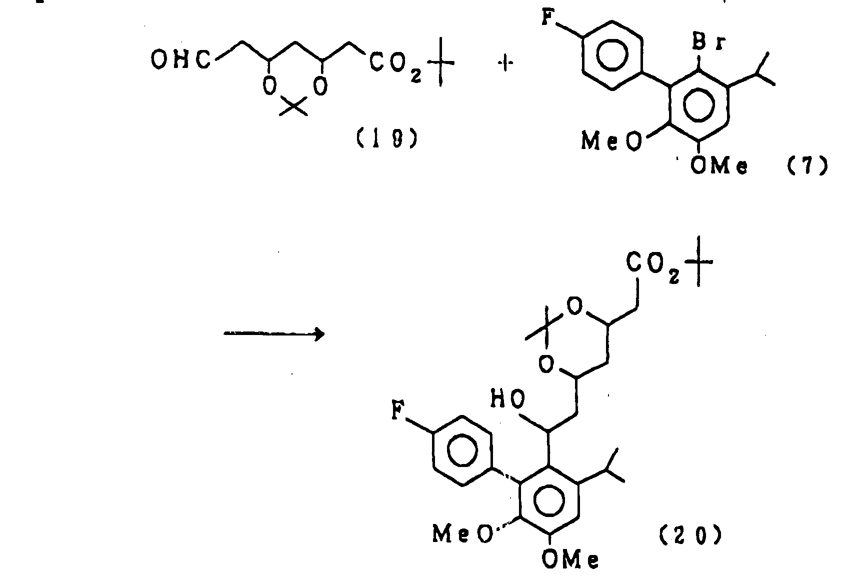

750 mg (2.11 mmol) of 2-bromo-4'-fluoro-5,6-dimethoxy-3-(propan-2-yl)biphenyl (compound (7)) and 213 mg (8.76 mmol) of magnesium turnings were added to 4 ml of anhydrous THF, and the mixture was refluxed in a stream of argon. To this suspension, a solution of 0.27 ml (2.10 mmol) of 1,2-dibromoethane in 4 ml of anhydrous THF was added dropwise over a period of 5 minutes. This reaction mixture was refluxed for 1 hour.

To this reaction mixture, a solution of 460 mg (1.69 mmol) of Compound (19) synthesized in Example 3 in 2 ml of anhydrous THF was added, and the mixture was stirred at room temperature for 1 hour.

This reaction mixture was then poured into a saturated aqueous solution of ammonium chloride, and the mixture was then extracted with ethyl acetate. The ethyl acetate extract layer was washed with a saturated aqueous solution of sodium chloride, dried over magnesium sulfate, and concentrated.

The residue was chromatographed on silica gel and eluted with a mixed solvent of hexane and ethyl acetate (2 : 1), whereby t-butyl 7-[4'-fluoro-5,6-dimethoxy-3-(propan-2-yl)-biphenyl-2-yl]-3,5-O-isopropylidene-3,5,7-trihydroxyheptanoate (compound (20)) was obtained in the form of a colorless amorphous solid in a yield of 670 mg (72.4 %).
Mixture of 1 : 1 stereoisomers
¹HNMR (CDCl₃, 300MHz)
δ 0.84 - 1.18 (m, 1H), 1.20 - 1.48 (m, 13H), 1.43 (s, 4.5H), 1.45 (s, 4.5H), 2.04 - 2.44 (m, 4H), 2.92 (d, J=1.6Hz, 0.5H), 3.46 (s, 1.5H), 3.47 (s, 1.5H), 3.89 (s, 3H), 3.70 - 4.25 (m, 3H), 4.68 - 4.78 (m, 0.5H), 4.87 - 4.97 (m, 0.5H), 6.91 (s, 1H), 7.00 -7.23 (m, 4H) ppm.
IR (KBr) ; 3524, 2960, 1732, 1590 cm⁻¹.
Mass (m/z, %) : 546 (M⁺, 36), 528 (20), 470 (28), 414 (69), 371 (28), 303 (100), 302 (49), 301 (39), 285 (77), 57 (32).

### Reference Example 16

### t-Butyl 7-[4'-fluoro-5,6-dimethoxy-3-(propan-2-yl)biphenyl-2-yl]-3,5-O-isopropylidene-6-hetenoate:

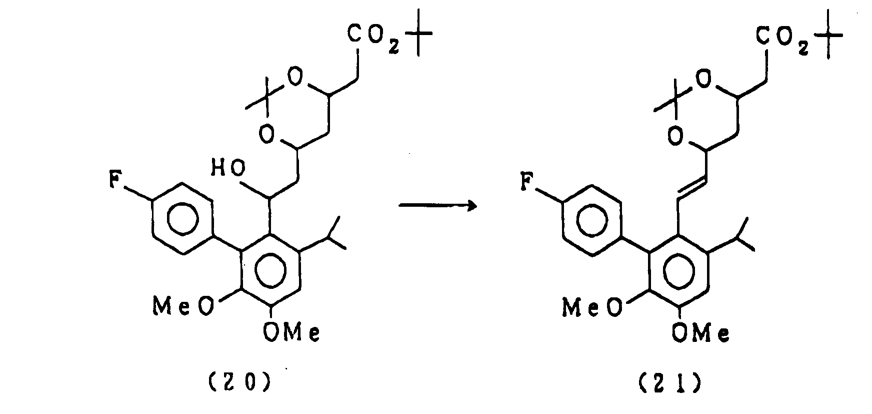

208 mg (0.38 mmol) of Compound (20) synthesized in Reference Example 15 was dissolved in 2.5 ml of 1,2-dichloroethane. To this solution, in a stream of argon at 0°C, 0.10 ml (1.24 mmol) of anhydrous pyridine and then 0.03 ml (0.41 mmol) of thionyl chloride were added, and the mixture was stirred for 1 hour.

This reaction mixture was poured into a saturated aqueous solution of sodium chloride, and the mixture was extracted with ethyl acetate.

The ethyl acetate extract layer was washed with an aqueous solution of sodium hydrogencarbonate and a saturated aqueous solution of sodium chloride, dried over magnesium sulfate, and then concentrated.

The residue was dissolved in 3 ml of 1,2-dichloroethane, and 0.10 ml (1.26 mmol) of anhydrous pyridine was added thereto. This mixture was refluxed in an atmosphere of argon for 2 hours.

This reaction mixture was then poured into a saturated aqueous solution of sodium chloride, and the mixture was then extracted with ethyl acetate. The ethyl acetate extract layer was washed with a saturated aqueous solution of sodium chloride, dried over magnesium sulfate, and concentrated.

The residue was chromatographed on silica gel and eluted with a mixed solvent of hexane and ethyl acetate (2 : 1), whereby t-butyl 7-[4'-fluoro-5,6-dimethoxy-3-(propan-2-yl)-biphenyl-2-yl]-3,5-O-isopropylidene-6- hetenoate (compound (21)) was obtained in a yield of 157 mg (78.0 %).
Melting point: 130.0 - 131.0°C (colorless needles, recrystallizzd from a mixed solvent of ethyl acetate and hexane)
¹HNMR (300MHz, CDCl₃)
δ 0.82 - 0.96 (m, 1H), 1.22 (d, J=6.8Hz, 3H), 1.23 (d, J=6.8Hz, 3H), 1.18 - 1.37 (m, 1H), 1.34 (s, 3H), 1.43 (s, 3H), 1.45 (s, 9H), 2.23 (dd, J=15.3 and 6.2Hz, 1H), 2.39 (dd, J=15.3 and 7.0Hz, 1H), 3.25 (hept, J=6.8Hz, 1H), 3.47 (s, 3H), 3.91 (s, 3H), 4.12 - 4.28 (m, 2H), 5.13 (dd, J=16.2 and 6.2Hz, 1H), 6.22 (dd, J=16.2 and 1.1Hz, 1H), 6.85 (s, 1H), 6.96 - 7.06 (m, 2H), 7.08 - 7.20 (m, 2H) ppm.
IR (KBr) : 2968, 1728, 1604, 1588 cm⁻¹.
Mass (m/z, %) : 528 (M⁺, 44), 470 (33), 415 (31), 414 (100), 397 (43), 371 (56), 326 (39), 311 (38), 298 (39), 285 (84), 283 (93).

## Claims

1. A heptanoate derivative of formula (I): 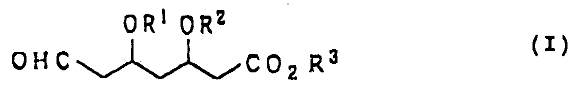 wherein R¹ and R² are independently a hydrogen atom or a protective group for the hydroxyl groups, or R¹ and R² taken together constitute a protective group for hydroxyl groups; and R³ is a hydrogen atom, an alkyl group, which may be substituted, or an aromatic hydrocarbon group.

2. The heptanoate derivative as claimed in Claim 1, wherein said protective group for hydroxyl group represented by R¹ or R² is selected from the group consisting of an alkyl group, an alkenyl group, a heterocyclic group, a silyl group having a substituent, an acyl group, an oxycarbonyl group having a substituent, and a carbamoyl group having a substituent; or said protective group formed integrally by R¹ and R² is selected from the group consisting of a methylene group, an alkylidene group and a carbonyl group.

3. The heptanoate derivative as claimed in Claim 2, wherein said alkyl group represented by R¹ or R² is an alkyl group having 1 to 6 carbon atoms, which may have a substituent.

4. The heptanoate derivative as claimed in Claim 3, wherein said substituent of said alkyl group represented by R¹ or R² is selected from the group consisting of an alkoxyl group having 1 to 6 carbon atoms, a phenyl group and naphthyl group.

5. The heptanoate derivative as claimed in Claim 2, wherein said alkenyl group represented by R¹ or R² is an alkenyl group having 2 to 6 carbon atoms, which may have a substituent.

6. The heptanoate derivative as claimed in Claim 5, wherein said substituent of said alkenyl group represented by R¹ or R² is a phenyl group.

7. The heptanoate derivative as claimed in Claim 2, wherein said heterocyclic group represented by R¹ or R² is selected from the group consisting of a 2-tetrahydropyranyl group, a 2-tetrahydrothiopyranyl group, and a 2-tetrahydrofuranyl group.

8. The heptanoate derivative as claimed in Claim 2, wherein said substituent of said silyl group represented by R¹ or R² is selected from the group consisting of an alkyl group having 1 to 6 carbon atoms, and a phenyl group.

9. The heptanoate derivative as claimed in Claim 2, wherein said acyl group represented by R¹ or R² is an acyl group having 1 to 6 carbon atoms, which may have a substituent.

10. The heptanoate derivative as claimed in Claim 9, wherein said substituent of said acyl group represented by R¹ or R² is selected from the group consisting of a halogen atom, an atkoxyl group having 1 to 6 carbon atoms, a phenoxy group and a phenyl group.

11. The heptanoate derivative as claimed in Claim 2, wherein said substituent of said oxycarbonyl group represented by R¹ or R² is selected from the group consisting of an alkyl group having 1 to 6 carbon atoms, an alkenyl group having 2 to 6 carbon atoms, and a phenyl group.

12. The heptanoate derivative as claimed in Claim 2, wherein said substituent of said carbamoyl group represented by R¹ or R² is selected from the group consisting of an alkyl group having 1 to 6 carbon atoms, a phenyl group, and a naphthyl group.

13. The heptanoate derivative as claimed in Claim 2, wherein said alkylidene group which is formed by R¹ and R² is an alkylidene group having 2 to 10 carbon atoms, which may have a substituent.

14. The heptanoate derivative as claimed in Claim 13, wherein said substituent of said alkylidene group which is formed by R¹ and R² is selected from the group consisting of halogen atom, an alkoxyl group having 1 to 6 carbon atoms, an amino group, and a phenyl group.

15. The heptanoate derivative as claimed in Claim 1, wherein said alkyl group represented by R³ is an alkyl group having 1 to 12 carbon atoms, which may have a substituent.

16. The heptanoate derivative as claimed in Claim 15, wherein said substituent of said alkyl group represented by R³ is selected from the group consisting of a phenyl group and a naphthyl group.

17. The heptanoate derivative as claimed in Claim 1, wherein said aromatic group represented by R³ is selected from the group consisting of a phenyl group and a naphthyl group.
